(19) Europäisches Patentamt
European Patent Office
Office européen des brevets

(11) Publication number: **0 460 687 A2**

(12) # EUROPEAN PATENT APPLICATION

(21) Application number: **91109331.8**

(51) Int. Cl.[5]: **C07C 65/24**, C07C 51/567

(22) Date of filing: **07.06.91**

(30) Priority: **08.06.90 US 535259**

(43) Date of publication of application:
**11.12.91 Bulletin 91/50**

(84) Designated Contracting States:
**AT BE CH DE DK ES FR GB GR IT LI LU NL SE**

(71) Applicant: **OCCIDENTAL CHEMICAL CORPORATION**
**2801 Long Road**
**Grand Island New York 14072-1244(US)**

(72) Inventor: **Stults, Jeffrey S.**
**1042 Baseline Road**
**Grand Island, New York 14072(US)**

(74) Representative: **Vossius & Partner**
**Siebertstrasse 4 P.O. Box 86 07 67**
**W-8000 München 86(DE)**

(54) **Process for the preparation of oxydiphthalic anhydride and acyloxyphthalic anhydrides.**

(57) A method for the preparation of acyloxyphthalic anhydride or oxydiphthalic anhydride which comprises the reaction of a halophthalic anhydride with an alkali metal salt and at least a catalytically effective amount of an acid or acid derivative selected from the group consisting of benzoic acids, substituted benzoic acids, benzoic acid salts or substituted benzoic acid salts, and hydrolyzable benzoyl esters, and hydrolyzable substituted benzoyl esters, alkyl carboxylic acids, hydrolyzable esters of alkyl carboxylic acids, and salts of alkyl carboxylic acids. The process may be conducted without solvent, in a polar solvent or in a non-polar solvent using a phase transfer catalyst.

EP 0 460 687 A2

This invention relates to a process for the preparation of acyloxyphthalic anhydrides, and oxydiphthalic anhydride from halophthalic anhydrides. More particularly, this invention relates to the use of benzoic acids, substituted benzoic acids, benzoic acid salts, substituted benzoic acid salts, benzoyloxyphthalic anhydrides, and substituted benzoyloxyphthalic anhydrides as catalysts for the reactions of a halophthalic anhydride to yield the compounds noted above.

Acyloxyphthalic anhydrides are particularly useful in the synthesis of oxydiphthalic anhydrides. By known methods the acyloxyphthalic anhydrides may be hydrolyzed to yield hydroxyphthalic anhydrides. The hydroxyphthalic anhydrides may be used to prepare oxydiphthalic anhydrides (US-A-4,837,404). The acyloxyphthalic anhydrides are also useful as curing agents for epoxy resins. The other product of this invention, oxydiphthalic anhydride is useful as a monomer for the preparation of polyimide resins. Polyimides may be produced by reacting an oxydiphthalic anhydride with a suitable diamine and dehydrating the resulting polyamic acid. Polyimide resins are particularly useful in electrical and high temperature applications.

Oxydiphthalic anhydride has been prepared by several methods.

US-A-4,697,023 discloses that a halophthalic anhydride, water, and an alkali metal compound such as potassium fluoride or potassium carbonate will react, in the presence of a dipolar aprotic solvent, to form oxydiphthalic anhydride.

US-A-4,837,404 teaches that oxydiphthalic anhydride may be prepared by reacting a halophthalic anhydride with an hydroxyphthalic anhydride in a polar aprotic solvent in the presence of an alkali metal compound such KF, CsF, or $K_2CO_3$.

US-A-4,808,731 teaches a method of preparing oxydiphthalic anhydride by reacting a substituted phthalic anhydride such as 4-nitro or 4-fluorophthalic anhydride with a dialkyl aminopyridine in the presence of a refluxing, non-polar organic solvent.

US-A-4,780,544 teaches a method of preparing oxy-di(N-alkyl or aryl) phthalimides. In this process nitro-N-alkyl or aryl phthalimide is treated with an alkali metal carboxylate salt. Sodium, potassium, and cesium salts of acetic acid, proprionic acid and benzoic acid are disclosed as suitable carboxylate salts and the effective amount is 0.25 to 1 mole of carboxylate per mole of nitrophthalimide.

Muehlemann (Pharm. Acta Helv. 23; 1948, p. 257) discloses the preparation 3-acetoxyphthalic acid from 3-hydroxyphthalic acid and acetic anhydride. Similarly, Japanese Patent application 85-238,404 as abstracted in CA 107 (22):199080g, discloses that 4-hydroxyphthalic anhydride biesters may be prepared by the condensation of 4-hydroxyphthalic anhydride with diacid chlorides. The biesters are formed from the diacid chloride and the hydroxy portion of the hydroxyphthalic anhydride. The anhydride portion of the molecule remains intact. German Patent DE-A-19 01 028 discloses that terphthalate esters of hydroxyphthalic anhydride may be prepared by the transesterification of 4-acetoxyphthalic anhydride with terphthalic acid in the presence of magnesium.

A method for the preparation of acyloxyphthalic anhydride or oxydiphthalic anhydride which comprises the reaction of a halophthalic anhydride with an alkali metal salt and at least a catalytically effective amount of an carboxylic acid or acid derivative including benzoic acid, substituted benzoic acids, benzoic acid salts or substituted benzoic acid salts, and hydrolyzable benzoyl esters, and hydrolyzable substituted benzoyl esters, alkyl carboxylic acids, hydrolyzable esters of alkyl carboxylic acids, and salts of alkyl carboxylic acids. The process may be conducted without solvent, in a polar solvent or in a non-polar solvent using a phase transfer catalyst.

The present invention relates to the reaction of halophthalic anhydrides with alkali metal salts, in a non-aqueous medium to form acyloxyphthalic anhydrides, or oxydiphthalic anhydride. The preferred alkali metal salts are potassium carbonate, potassium fluoride, cesium fluoride, or sodium carbonate.

In the absence of a catalyst, the reaction of halophthalic anhydride with an alkali metal salt, such as potassium carbonate, is slow. Comparative examples 1 and 2 illustrated such reactions. Surprisingly, it has now been found that small catalytic quantities of a carboxylic acid or acid derivative catalyze the reaction between a halophthalic anhydride and an alkali metal salt in a non-aqueous medium. The preferred acid or acid derivatives are benzoic acid, substituted benzoic acids, benzoic acid salts, substituted benzoic acid salts, hydrolyzable benzoyl esters, and hydrolyzable substituted benzoyl esters, alkyl carboxylic acids, hydrolyzable esters of alkyl carboxylic acids, and salts of alkyl carboxylic acids.

Hereinafter, the term acid or acid derivatives means a carboxylic acid or derivatives thereof, benzoic acid, substituted benzoic acids, benzoic acid salts, substituted benzoic acid salts, hydrolyzable benzoyl esters including esters of benzoic acids and substituted benzoic acids, alkyl carboxylic acids, hydrolyzable esters of alkyl carboxylic acids, or salts of alkyl carboxylic acids. The term alkyl carboxylic acids includes $C_2$ to $C_{12}$ straight, branched chain, and cyclic acids. Most esters of benzoic acids, or alkyl carboxylic acids are hydrolyzable under the conditions of this reaction. However, esters with steric hindrance near the

carboxyl group, or other unusual structural features may not hydrolyze readily enough to be useable as catalysts for the reaction. Those skilled in the art will appreciate which esters are suitable as catalysts. Benzoyloxyphthalic anhydride or a substituted benzoyloxyphthalic anhydrides such as 4-(chlorobenzoyloxy)phthalic anhydride, and simple esters such as ethyl acetate, and ethyl ethyl benzoate are suitable catalysts for use in the present invention. The selected catalyst should not contain substituents which interfere with the reaction of this invention. In addition, if a low boiling ester or alkyl carboxylic acid is selected as the catalyst, steps must be taken to prevent its loss during the course of the reaction. A wide variety of benzoate salts and alkyl carboxylate salts are suitable for use as catalysts. However, the Li, Na, K, Cs and $NH_4+$ salts are preferred. A wide variety of substituted benzoic acids are catalytically effective. However, the preferred substituents are nitro, halo, dihalo, trihalo, cyano and trihalomethyl. The preferred akyl carboxylic acids are the $C_2$ to $C_6$ alkyl carboxylic acids including branched chain and cyclic isomers.

A non-aqueous medium means either a non-aqueous solvent, or no solvent. If no solvent is used, the halophthalic anhydride itself acts as the solvent. The effective quantity of the catalyst is less than about 1 mole percent based upon the quantity of halophthalic anhydride.

The reaction of a halophthalic anhydride with an alkali metal salt, in the presence of the acid or acid derivative, produces two products, acyloxyphthalic anhydrides, and oxydiphthalic anhydride:

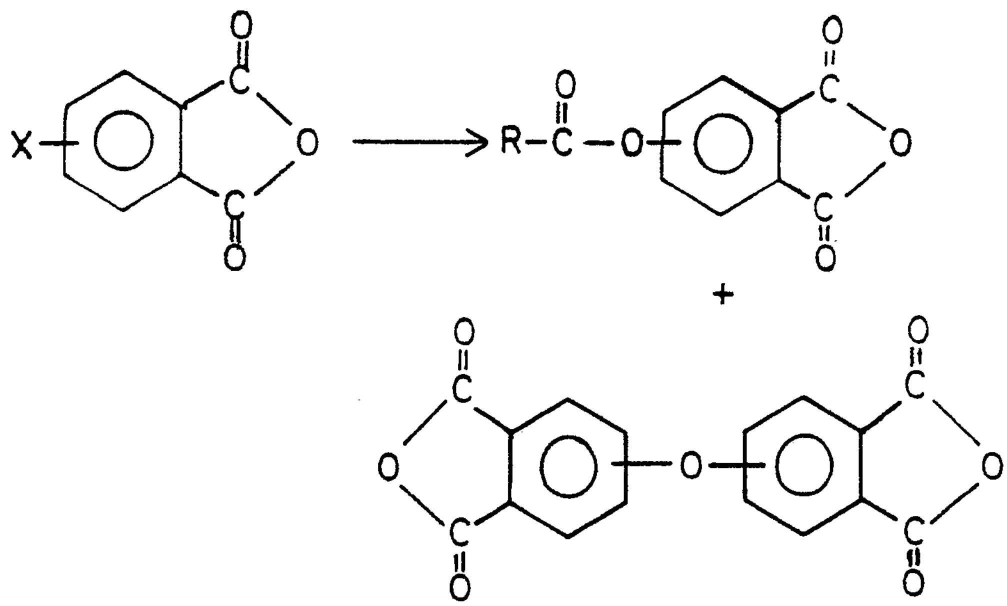

where x = F, Cl, Br, or I
and R is phenyl, substituted phenyl, and $C_2$ to $C_{12}$ alkyl.

As set forth more fully below, the reaction conditions can be varied to emphasize one product at the expense of the other. For example, the catalyst can take two different roles in the reaction of this invention. If the selected catalyst is present in small quantities, it acts only as a catalyst for the reaction, and the reaction produces oxydiphthalic anhydride. On the other hand, if the concentration of the catalyst is higher, the catalyst becomes a reactant as well, and an acyloxyphthalic anhydride is an observed product. If the catalyst is present in large enough concentrations to act as a reactant, low temperature reactions tend to favor the production of a acyloxyphthalic anhydrides. Higher temperatures favor the formation of oxydiphthalic anhydride.

The process of this invention applies to a wide variety of halophthalic anhydrides. The halogen substituent on the starting halophthalic anhydride may be fluorine, chlorine, bromine or iodine. The preferred halo substituents are fluorine and chlorine. Both the 3-halo and 4-halo phthalic anhydride may be used in this reaction. Accordingly, it is possible, using the method of this invention, to prepare 3-acyloxyphthalic anhydrides, 4-acyloxyphthalic anhydrides, 3,3'-oxydiphthalic anhydride, 4,4'-oxydiphthalic anhydride and 3,4'-oxydiphthalic anhydride.

The reaction may be conducted without solvent, or in a wide variety of solvents. If the reaction is run without a solvent, care must be taken to avoid overheating which can cause color forming side reactions. If the reaction is run in a solvent, it is preferred to use solutions of high concentration. Typical concentrations are 1:1 by weight. The reaction may be conducted in more dilute solutions. However, the rate of the

reaction may be slower in such solutions, and the solvent removal on workup may be more difficult. Solvents containing a reactive group such as an hydroxyl or a primary or secondary amine are not suitable. Polar, aprotic solvents such as dimethyl sulfoxide, sulfolane, dimethylsulfone, N,N-dimethyl formamide, hexamethylphosphoramide, N-methyl pyrrolidone, and dimethyl acetamide are suitable for this process. Less polar solvents of a moderately high boiling point, such as bromobenzene, chlorobenzene, dichlorobenzenes, trichlorobenzenes, benzonitrile, dichlorotoluenes, and chloroxylenes may also be used. The preferred solvent is 1,2,4-trichlorobenzene. Less polar solvents of lower boiling point, such as toluene, may be used provided that the reaction is conducted under sufficient pressure to achieve the desired temperature. In polar solvents, the reactants are sufficiently soluble and a phase transfer catalyst is not required. In less polar solvents, it is preferred to use phase transfer catalysts to increase the rate of the reaction.

Phase transfer catalysts combine, within the same molecule, both polar and non-polar regions. The non-polar regions of the phase transfer catalyst allow it to dissolve in non-polar solvents. On the other hand, the polar regions of the molecule allow for interaction with more polar species. Accordingly, a phase transfer catalyst can affect the solubility of a polar species in a non-polar solvent. In the absence of a catalyst, a polar species, such as a benzoate ion, might be rather insoluble in a non-polar solvent. In the presence of a phase transfer catalyst, the ion can exist in a non-polar solvent in appreciable concentrations. A wide variety of phase transfer catalysts will function in allowing this process to function in non-polar solvents. Among the phase transfer catalysts which are useful in this invention are tetraalkyl ammonium salts, n-alkyl pyridinium salts, tetraalkyl phosphonium salts, crown ethers, polyethers, and tetraphenyl phosphonium salts. The choice of phase transfer catalysts depends on the temperature at which the reaction is to be conducted. For example, if the reaction can be conducted at 150° C or less, tetra alkyl ammonium salts are useable. On the other hand, at higher temperatures more stable catalysts such as tetraphenyl phosphonium salts are preferred. The most preferred phase transfer catalysts are tetraphenyl phosphonium benzoates, tetraphenyl phosphonium substituted benzoates, and tetraphenylphosphonium halides. Among the substituted benzoates which may be used in such catalysts are nitrobenzoates, halobenzoates, alkylbenzoates, and alkoxybenzoates.

The temperature at which the reaction is conducted depends upon the halophthalic anhydride chosen as a starting material. If a fluorophthalic anhydride is selected as the starting material, the reaction may be run between about 120° and 250° C. Chlorophthalic anhydride requires a reaction temperature of about 170° to 275° C. Bromophthalic anhydrides fall between fluoro and chlorophthalic anhydrides in temperature requirements.

The time of reaction is highly dependent both upon the temperature, and upon the concentration of solution. As with most chemical reactions, the reaction is over more quickly at higher temperatures; however, side reactions become more available at the higher temperatures. Similarly, the reaction is rapid in solutions of high concentrations. However, reactions run in more concentrated solutions may suffer from side reactions. The reaction may be run at a concentration of 2 parts solvent to one part halophthalic anhydride starting material or lower concentrations of the halophthalic anhydride. In selecting appropriate conditions, one must balance speed of reaction and purity of the final product.

The reaction may be run to completion. However, it is often found that when the reaction is run to completion, colored side products are formed. Accordingly, it is often desirable to limit the amount of basic salt used in the reaction, and to limit the reaction time so that the reaction goes only part way to completion. Fractional crystallization will readily separate the reaction product oxydiphthalic anhydride from the halophthalic anhydride starting material which may be readily recovered and recycled. Depending upon the source, the halophthalic anhydride starting material may contain impurities which inhibit the reaction. Although the nature of these impurities is not entirely certain, their effect may be controlled by heating the halophthalic anhydride with the basic salt prior to the addition of the catalyst.

The kinetics of this reaction are rather complex. The role of the catalyst is not simple. The carboxylic acid, alkyl carboxylic acid or benzoic acid derivative which produces the best results, as a catalyst, varies depending upon the reaction conditions. It has been observed that in non-polar solvents, the more acidic derivatives of benzoic acid, such as nitrobenzoic acid, dinitrobenzoic acid, halobenzoic acids, dihalobenzoic acids, trihalobenzoic acids, cyanobenzoic acids and trihalomethyl benzoic acids and the more acidic alkyl carboxylic acids tend to be more effective as catalysts. On the other hand, benzoic acid, alkylbenzoic acids, alkoxybenzoic acids or their salts as well as the less acid alkyl carboxylic acids are very effective catalysts in more polar solvents. In the preferred solvent, 1,2,4-trichlorobenzene, monohalobenzoic acids are the preferred catalysts. For any given solvent, the preferred catalyst may easily be determined by conducting two or three small scale experiments with catalysts of different acidity.

The role of water in the reaction is unusual. Some water is necessary for the reaction to begin. If the potassium carbonate is used as the basic salt, water is produced when the basic salt scavenges the acid

produced by the displacement of the halogen from the halophthalic anhydride. However, excessive amounts of water are undesirable because water can react with the phthalic anhydride to form phthalic acid. This reaction reduces the yield of the desired product. It has been found that when the basic salt is potassium carbonate, and reaction is run in a solvent, it is preferable that the solvent contain approximately 0.05 to 0.2 mole% total water based upon the halophthalic anhydride in order for the reaction to proceed appropriately. On the other hand, it has been found that if the total water in the solvent is 0.5 mole%, or above,based upon the halophthalic anhydride the yield of product is decreased.

If the reaction is conducted with potassium carbonate in a polar, aprotic solvent, there is generally sufficient water present in the solvent so that the reaction will proceed. Alternatively, water is often present in commercial samples of alkali metal salts such as potassium carbonate. Accordingly, there is generally sufficient water present from either the salt or the solvent so that the reaction can be conducted even in a relatively dry, non-polar solvent. If the reaction is to be conducted with truly dry materials, then an appropriate quantity of water must be provided for the reaction to begin. However, excess water is to be avoided since it can lead to hydrolysis of the phthalic anhydride and, therefore, a reduction in yield of product.

When potassium fluoride or cesium fluoride is used as the basic salt, the role of water is somewhat different. Here water is not produced during the reaction, and some water must be provided in order for the reaction to occur. Depending upon which product is desired, one may readily calculate how much water is required. For example, if the desired product is the acyloxyphthalic anhydride, no additional water is required. However, if the desired product is the oxydiphthalic anhydride, one half mole of water is required for each mole of halophthalic anhydride starting material. If sufficient water is not present in the solvent or in the reactants, it may be necessary to add some water to the reaction mixture in order to obtain the desired product. It is preferred to add the water incrementally throughout the reaction in order to avoid the presence of excessive quantities of water which can lead to hydrolysis of the phthalic anhydride.

The process of the present invention provides a novel route to the synthesis of a wide variety of acyloxyphthalic anhydrides. Among the novel compounds that may be produced by the process of the invention are acyloxyphthalic anhydrides of the formulas

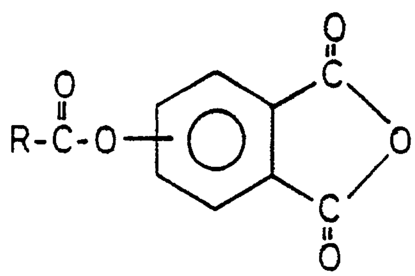

where R is $C_2H_5$, $C_3H_7$, $C_4H_9$, $C_5H_{11}$, $C_6H_{13}$, $C_7H_{15}$, $C_8H_{17}$, $C_9H_{19}$, $C_{10}H_{21}$, or $C_{11}H_{23}$, including branched and cyclic isomers of those formulas; and

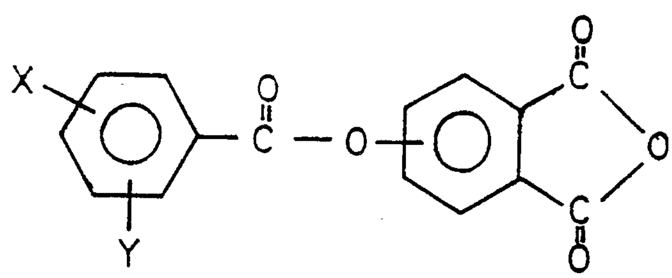

where X and Y are substituents independently selected from H, F, Cl, Br, $NO_2$, $CH_3$, $CF_3$, $CCl_3$ and CN, provided that X and Y may not both be H.

Without further elaboration, it is believed that one skilled in the art can, using the preceding description, utilize the present invention to its fullest extent. The following specific embodiments are, therefore, to be construed as merely illustrative, and not limiting, in any way, the remainder of the disclosure. In the following examples, all temperatures are set forth uncorrected in degrees Celsius and parts and percentages are by weight, unless otherwise indicated.

Example 1

4-Chlorophthalic anhydride (30 g, 160 mmol) and benzonitrile (Aldrich, 30 g, containing 110 ppm water) were heated to reflux. Dry potassium carbonate (1.2 g, 8.7 mmol) was added and the mixture heated for 30 minutes. Tetraphenylphosphonium bromide (0.36 g, 0.9 mmol) and 4-chlorobenzoic acid (0.06 g, 0.4 mmol) were added followed by addition of potassium carbonate (11.4 g, 82.2 mmol). The reaction was heated for about 9 hours and then was diluted with benzonitrile (30 g) and filtered. The 4,4'-oxydiphthalic anhydride was allowed to crystallize, was washed with solvent, and dried in an air circulating oven at 120° C to give crude 4,4'-oxydiphthalic anhydride, 24.7 g.

Example 2

4-Chlorophthalic anhydride (30 g, 160 mmol) and 1,2-dichlorobenzene (Aldrich, 30 g) were heated to reflux. Dry potassium carbonate (1.04 g, 7.5 mmol) was added and the mixture heated for 30 minutes. Tetraphenylphosphonium bromide (0.36 g, 0.9 mmol) and 4-chlorobenzoic acid (0.06 g, 0.4 mmol) were added followed by addition of potassium carbonate (11.4 g, 82.2 mmol). The reaction was heated for about 10 hours and then was diluted with 1,2-dichlorobenzene (60 g) and filtered. The 4,4'-oxydiphthalic anhydride was allowed to crystallize, was washed with solvent, and dried in an air circulating oven at 120° C to give crude 4,4'-oxydiphthalic anhydride, 22.3 g.

Example 3

4-Chlorophthalic anhydride (30 g, 160 mmol) and 2,4-dichlorotoluene (Aldrich, 30 g) were heated to reflux at approximately 218 °C. Dry potassium carbonate (1.32 g, 12 mmol) was added and the mixture heated for about 30 minutes. Tetraphenylphosphonium bromide (0.36 g, 0.9 mmol) and 4-chlorobenzoic acid (0.06 g, 0.4 mmol) were added followed by addition of potassium carbonate (11.4 g, 82.2 mmol) over 45 minutes. The reaction was heated for about 6 hours and then was diluted with 2,4-dichlorotoluene (60 g) and filtered. The 4,4'-oxydiphthalic anhydride was allowed to crystallize, was washed with solvent, and dried in an air circulating oven at 120° C to give crude 4,4'-oxydiphthalic anhydride, 20.3 g.

Example 4

4-Chlorophthalic anhydride (30 g, 160 mmol) and 2,6-dichlorotoluene (Aldrich, 15 g) were heated to reflux at approximately 221 °C. Dry potassium carbonate (1.26 g, 9.1 mmol) was added and the mixture heated for about 1 hour. Tetraphenylphosphonium bromide (0.36 g, 0.9 mmol) and 4-chlorobenzoic acid (0.06 g, 0.4 mmol) were added followed by addition of potassium carbonate (11.4 g, 82.2 mmol). The reaction was heated for about 6.5 hours and then was diluted with 2,6-dichlorotoluene (60 g) and filtered. The 4,4'-oxydiphthalic anhydride was allowed to crystallize, was washed with solvent, and dried in an air circulating oven at 120° C to give crude 4,4'-oxydiphthalic anhydride, 23 g.

Example 5

4-Chlorophthalic anhydride (30 g, 160 mmol) and 2,5-dichlorotoluene (Aldrich, 15 g) were heated to reflux at approximately 230 °C. Dry potassium carbonate (1.26 g, 9.1 mmol) was added and the mixture heated for about 1 hour. Tetraphenylphosphonium bromide (0.36 g, 0.9 mmol) and 4-chlorobenzoic acid (0.06 g, 0.4 mmol) were added followed by addition of potassium carbonate (11.4 g, 82.2 mmol). The reaction was heated for about 5 hours and then was diluted with 2,5-dichlorotoluene (60 g) and filtered. The 4,4'-oxydiphthalic anhydride was allowed to crystallize, was washed with solvent, and dried in an air circulating oven at 120° C to give crude 4,4'-oxydiphthalic anhydride, 20.9 g.

Example 6

4-Chlorophthalic anhydride (30 g, 160 mmol) and mixed dichlorotoluenes (15 g) were heated to. Dry potassium carbonate (1.29 g, 9.3 mmol) was added and the mixture heated for about 1 hour. Tetraphenylphosphonium bromide (0.36 g, 0.9 mmol) and 4-chlorobenzoic acid (0.06 g, 0.4 mmol) were added followed by addition of potassium carbonate (11.4 g, 82.2 mmol). The reaction was heated for about 10 hours and then was diluted with mixed dichlorotoluenes (60 g) and filtered. The 4,4'-oxydiphthalic anhydride was allowed to crystallize, was washed with solvent, and dried in an air circulating oven at 120° C to give crude

4,4'-oxydiphthalic anhydride, 18.4 g.

Example 7

3-Chlorophthalic anhydride (30.1 g, 160 mmol) and 1,2,4-trichlorobenzene (30.5 g) were heated to reflux. Dry potassium carbonate (1.17 g, 8.5 mmol) was added and the suspension heated for 1 hour. Tetraphenylphosphonium bromide (0.36 g, 0.9 mmol) and 4-chlorobenzoic acid (0.06 g, 0.4 mmol) was added followed by addition of more potassium carbonate (11.36 g, 82.2 mmol). The reaction was heated for approximately 12 hours. The reaction mixture was diluted with 1,2,4-trichlorobenzene (60 ml), filtered, and 3,3'-oxydiphthalic anhydride allowed to crystallize. The solid was collected, washed with solvent, and dried in an air circulating oven at 120° C to give crude 3,3'-oxydiphthalic anhydride, 19.6 g.

Example 8

4-Chlorophthalic anhydride (10 g, 55 mmol), potassium benzoate (1.0 g, 6.2 mmol), and sulfolane (25 g) were heated to about 162° C. Potassium carbonate (3.87 g, 28 mmol) was added and the suspension was heated to about 175° C. After 4 hours, GC analysis indicated a 6:3 ratio of 4,4'-oxydiphthalic anhydride to 4-chlorophthalic anhydride.

Example 9

4-Chlorophthalic anhydride (10 g, 55 mmol), potassium benzoate (1.0 g, 6.2 mmol), and N,N-dimethylformamide (25 g) were heated to reflux. Potassium carbonate (3.87 g, 28 mmol) was added. After about 6.5 hours, GC analysis indicated a 2:1 ratio of 4,4'-oxydiphthalic anhydride to 4-chlorophthalic anhydride.

Example 10

4-Chlorophthalic anhydride (10 g, 55 mmol), potassium benzoate (1.0 g, 6.2 mmol), and N-methylpyrrolidinone (25 g) were heated to about 175° C. Potassium carbonate (3.87 g, 28 mmol) was added and the suspension was heated to about 175° C. After 2 hours, GC analysis indicated a greater than 97% conversion to 4,4'-oxydiphthalic anhydride. The solvent was removed under reduced pressure and the residue treated with an aqueous 25% sodium hydroxide solution. The solution was acidied with concentrated HCl. The solid was collected and dried to give 4,4'-oxydiphthalic acid (4.85 g) mixed with some sodium chloride. The tetra acid was cyclized and recrystallized from hot acetic anhydride (2.4 mls) and filtered to remove salts to give 4,4'-oxydiphthalic anhydride, 4.2 g.

Example 11

4-Chlorophthalic anhydride (30 g, 160 mmol), and nitrobenzene (Aldrich, 30 g, containing 500 ppm water) were heated to reflux. Dry potassium carbonate (1.32 g, 9.6 mmol) was added and the mixture heated for 30 minutes. Tetraphenylphosphonium bromide (0.36 g, 0.9 mmol) and 4-chlorobenzoic acid (0.06 g, 0.4 mmol) were added followed by addition of potassium carbonate (11.4 g, 82.2 mmol). The reaction was heated for about 9 hours and then was diluted with nitrobenzene (30 g) and filtered. The 4,4'-oxydiphthalic anhydride was allowed to crystallize, was washed with solvent, and dried in an air circulating oven at 120° C to give crude 4,4'-oxydiphthalic anhydride, 10.0 g.

Example 12

4-Chlorophthalic anhydride (30 g, 160 mmol) and 1,2,4-trichlorobenzene (30 g) were heated to reflux (approximately 224° C) and tetraphenylphosphonium bromide (Hokko Chemical, 0.30 g, 0.7 mmol) and 4-chlorobenzoic acid (Aldrich, 0.06 g, 0.4 mmol) were added. Dry potassium carbonate (7.95 g, 58 mmol) was then added in portions over one hour. The reaction was monitored by GC and showed 62.9% conversion to 4,4'-oxydiphthalic anhydride (ODPAN) 3 hours after addition of carbonate was completed. The reaction was heated for 3.5 hours after addition of potassium carbonate and was then diluted with 1,2,4-trichlorobenzene (60 g), filtered, and the ODPAN allowed to recrystallize. The ODPAN was collected and washed with 1,2,4-trichlorobenzene followed by hexanes and dried in an air circulating oven at 120° C to give crude ODPAN, 16.4 g (100% based on conversion of 4-chlorophthalic anhydride).

Example 13

4-Chlorophthalic anhydride (30 g, 160 mmol) and 1,2,4-trichlorobenzene (30 g) were heated to reflux (approximately 224°C) and tetraphenylphosphonium bromide (Hokko Chemical, 0.30 g, 0.7 mmol) and 3-bromobenzoic acid (Aldrich, 0.06 g, 0.4 mmol) were added. Dry potassium carbonate (7.95 g, 58 mmol) was then added in portions over one hour. The reaction was monitored by GC and showed 61.2% conversion to 4,4'-oxydiphthalic anhydride (ODPAN) 3 hours after addition of carbonate was completed. The reaction was heated for 3.5 hours after addition of potassium carbonate and was then diluted with 1,2,4-trichlorobenzene (60 g), filtered, and the ODPAN allowed to recrystallize. The ODPAN was collected and washed with 1,2,4-trichlorobenzene followed by hexanes and dried in an air circulating oven at 120°C to give crude ODPAN, 16.4 g (100% based on conversion of 4-chlorophthalic anhydride).

Example 14

4-Chlorophthalic anhydride (30 g, 160 mmol) and 1,2,4-trichlorobenzene (60 g) were heated to reflux (approximately 224°C) and tetraphenylphosphonium bromide (Hokko Chemical, 0.30 g, 0.7 mmol) and 3,4-dichlorobenzoic acid (Aldrich, 0.06 g, 0.4 mmol) were added. Dry potassium carbonate (7.95 g, 58 mmol) was then added in portions over one hour. The reaction was monitored by GC and showed 48% conversion to 4,4'-oxydiphthalic anhydride (ODPAN) 3.5 hours after addition of carbonate was completed. The reaction was heated for 6 hours after addition of potassium carbonate and was then diluted with 1,2,4-trichlorobenzene (30 g), filtered, and the ODPAN allowed to recrystallize. The ODPAN was collected and washed with 1,2,4-trichlorobenzene followed by hexanes and dried in an air circulating oven at 120°C to give crude ODPAN, 13.8 g (89% based on conversion of 4-chlorophthalic anhydride).

Example 15

4-Chlorophthalic anhydride (30 g, 160 mmol) and 1,2,4-trichlorobenzene (60 g) were heated to reflux (approximately 224°C) and tetraphenylphosphonium bromide (Hokko Chemical, 0.30 g, 0.7 mmol) and 2,4-dichlorobenzoic acid (Aldrich, 0.09 g, 0.4 mmol) were added. Dry potassium carbonate (7.95 g, 58 mmol) was then added in portions over one hour. The reaction was monitored by GC and showed 47.2% conversion to 4,4'-oxydiphthalic anhydride (ODPAN) 4 hours after addition of carbonate was completed. The reaction was heated for 7 hours after addition of potassium carbonate and was then diluted with 1,2,4-trichlorobenzene (30 g), filtered, and the ODPAN allowed to recrystallize. The ODPAN was collected and washed with 1,2,4-trichlorobenzene followed by hexanes and dried in an air circulating oven at 120°C to give crude ODPAN, 12.96 g (100% based on conversion of 4-chlorophthalic anhydride).

Example 16

4-Chlorophthalic anhydride (30 g, 160 mmol) and 1,2,4-trichlorobenzene (60 g) were heated to reflux (approximately 224°C) and tetraphenylphosphonium bromide (Hokko Chemical, 0.30 g, 0.7 mmol) and 2-chlorobenzoic acid (Aldrich, 0.06 g, 0.4 mmol) were added. Dry potassium carbonate (7.95 g, 58 mmol) was then added in portions over one hour. The reaction was monitored by GC and showed 14.9% conversion to 4,4'-oxydiphthalic anhydride (ODPAN) 4 hours after addition of carbonate was completed.

Example 17

4-Chlorophthalic anhydride (30 g, 160 mmol) and 1,2,4-trichlorobenzene (60 g) were heated to reflux (approximately 224°C) and tetraphenylphosphonium bromide (Hokko Chemical, 0.30 g, 0.7 mmol) and 4-bromobenzoic acid (Aldrich, 0.09 g, 0.4 mmol) were added. Dry potassium carbonate (7.95 g, 58 mmol) was then added in portions over one hour. The reaction was monitored by GC and showed 49.6% conversion to 4,4'-oxydiphthalic anhydride (ODPAN) 3 hours after addition of carbonate was completed. The reaction was heated for 3.5 hours after addition of potassium carbonate and was then diluted with 1,2,4-trichlorobenzene (30 g), filtered, and the ODPAN allowed to recrystallize. The ODPAN was collected and washed with 1,2,4-trichlorobenzene followed by hexanes and dried in an air circulating oven at 120°C to give crude ODPAN, 14.7 g (100% based on conversion of 4-chlorophthalic anhydride).

Example 18

4-Chlorophthalic anhydride (30 g, 160 mmol) and 1,2,4-trichlorobenzene (60 g) were heated to reflux (approximately 224° C) and tetraphenylphosphonium bromide (Hokko Chemical, 0.30 g, 0.7 mmol) and 3,5-dichlorobenzoic acid (Aldrich, 0.09 g, 0.4 mmol) were added. Dry potassium carbonate (7.95 g, 58 mmol) was then added in portions over one hour. The reaction was monitored by GC and showed 31.9% conversion to 4,4'-oxydiphthalic anhydride (ODPAN) 3 hours after addition of carbonate was completed. The reaction was heated for 7 hours after addition of potassium carbonate and was then diluted with 1,2,4-trichlorobenzene (30 g), filtered, and the ODPAN allowed to recrystallize. The ODPAN was collected and washed with 1,2,4-trichlorobenzene followed by hexanes and dried in an air circulating oven at 120° C to give crude ODPAN, 12.8 g (100% based on conversion of 4-chlorophthalic anhydride).

Example 19

4-Chlorophthalic anhydride (30 g, 160 mmol) and 1,2,4-trichlorobenzene (60 g) were heated to reflux (approximately 224° C) and tetraphenylphosphonium bromide (Hokko Chemical, 0.30 g, 0.7 mmol) and 3-chlorobenzoic acid (Aldrich, 0.06 g, 0.4 mmol) were added. Dry potassium carbonate (7.95 g, 58 mmol) was then added in portions over one hour. The reaction was monitored by GC and showed 26.7% conversion to 4,4'-oxydiphthalic anhydride (ODPAN) 4 hours after addition of carbonate was completed. The reaction was heated for 11.5 hours after addition of potassium carbonate and was then diluted with 1,2,4-trichlorobenzene (30 g), filtered, and the ODPAN allowed to recrystallize. The ODPAN was collected and washed with 1,2,4-trichlorobenzene followed by hexanes and dried in an air circulating oven at 120° C to give crude ODPAN, 10.22 g (100% based on conversion of 4-chlorophthalic anhydride).

Example 20

4-Chlorophthalic anhydride (30 g, 160 mmol) and 1,2,4-trichlorobenzene (60 g) were heated to reflux (approximately 224° C) and tetraphenylphosphonium bromide (Hokko Chemical, 0.30 g, 0.7 mmol) and 4-methoxybenzoic acid (Aldrich, 0.06 g, 0.4 mmol) were added. Dry potassium carbonate (7.95 g, 58 mmol) was then added in portions over one hour. The reaction was monitored by GC and showed 3.7% conversion to 4,4'-oxydiphthalic anhydride (ODPAN) 4 hours after addition of carbonate was completed.

Example 21

4-Chlorophthalic anhydride (30 g, 160 mmol) and 1,2,4-trichlorobenzene (60 g) were heated to reflux (approximately 224° C) and tetraphenylphosphonium bromide (Hokko Chemical, 0.30 g, 0.7 mmol) and 4-methylbenzoic acid (Aldrich, 0.05 g, 0.4 mmol) were added. Dry potassium carbonate (7.95 g, 58 mmol) was then added in portions over one hour. The reaction was monitored by GC and showed 3.7% conversion to 4,4'-oxydiphthalic anhydride (ODPAN) 4 hours after addition of carbonate was completed.

Example 22

4-Chlorophthalic anhydride (30 g, 160 mmol) and 1,2,4-trichlorobenzene (60 g) were heated to reflux (approximately 224° C) and tetraphenylphosphonium bromide (Hokko Chemical, 0.30 g, 0.7 mmol) and 3-nitrobenzoic acid (Aldrich, 0.07 g, 0.4 mmol) were added. Dry potassium carbonate (7.95 g, 58 mmol) was then added in portions over one hour. The reaction was monitored by GC and showed 12.5% conversion to 4,4'-oxydiphthalic anhydride (ODPAN) 4 hours after addition of carbonate was completed. The reaction was heated for 11.5 hours after addition of potassium carbonate and was then diluted with 1,2,4-trichlorobenzene (30 g), filtered, and the ODPAN allowed to recrystallize. The ODPAN was collected and washed with 1,2,4-trichlorobenzene followed by hexanes and dried in an air circulating oven at 120° C to give crude ODPAN, 4.52 g (100% based on conversion of 4-chlorophthalic anhydride).

Example 23

4-Chlorophthalic anhydride (30 g, 160 mmol) and 1,2,4-trichlorobenzene (60 g) were heated to reflux (approximately 224° C) and tetraphenylphosphonium bromide (Hokko Chemical, 0.30 g, 0.7 mmol) and 4-nitrobenzoic acid (Aldrich, 0.07 g, 0.4 mmol) were added. Dry potassium carbonate (7.95 g, 58 mmol) was then added in portions over one hour. The reaction was monitored by GC and showed 23.1% conversion to 4,4'-oxydiphthalic anhydride (ODPAN) 4 hours after addition of carbonate was completed. The reaction was heated for 11.5 hours after addition of potassium carbonate and was then diluted with 1,2,4-trichlorobenzene

(30 g), filtered, and the ODPAN allowed to recrystallize. The ODPAN was collected and washed with 1,2,4-trichlorobenzene followed by hexanes and dried in an air circulating oven at 120° C to give crude ODPAN, 9.49 g (100% based on conversion of 4-chlorophthalic anhydride).

Example 24

4-Chlorophthalic anhydride (30 g, 160 mmol), tetraphenylphosphonium bromide (0.3 g, 0.7 mmol), and 4-chlorobenzoic acid (0.06 g, 0.4 mmol) were heated to approximately 220° C. Potassium carbonate (7.95 g, 58 mmol) was added over 45 minutes. The reaction was sampled after an additional 30 minutes and showed a 68.3% conversion to 4,4'-oxydiphthalic anhydride. The reaction mixture was diluted with 1,2,4-trichlorobenzene (90 g) and filtered. The 4,4'-oxydiphthalic anhydride was allowed to crystallize and was collected, washed with 1,2,4-trichlorobenzene followed by hexane, and dried in an air circulating oven at 120° C to give crude 4,4'-oxydiphthalic anhydride, 15.6 g (88% yield based on conversion).

Example 25

4-Chlorophthalic anhydride (30 g, 160 mmol), tetraphenylphosphonium bromide (0.3 g, 0.7 mmol), and 2,5-dichlorobenzoic acid (0.08 g, 0.4 mmol) were heated to approximately 220° C. Potassium carbonate (7.95 g, 58 mmol) was added over 45 minutes. The reaction was sampled after addition of the potassium carbonate and showed a 24.9% conversion to 4,4'-oxydiphthalic anhydride. The reaction was heated for an additional 45 minutes. The reaction mixture was diluted with 1,2,4-trichlorobenzene (90 g) and filtered. The 4,4'-oxydiphthalic anhydride was allowed to crystallize and was collected, washed with 1,2,4-trichlorobenzene followed by hexane, and dried in an air circulating oven at 120° C to give crude 4,4'-oxydiphthalic anhydride, 12.9 g.

Example 26

4-Chlorophthalic anhydride (30 g, 160 mmol), tetraphenylphosphonium bromide (0.3 g, 0.7 mmol), and 2,6-dichlorobenzoic acid (0.08 g, 0.4 mmol) were heated to approximately 220° C. Potassium carbonate (7.95 g, 58 mmol) was added over 45 minutes. The reaction was sampled after addition of the potassium carbonate and showed a 28.3% conversion to 4,4'-oxydiphthalic anhydride. The reaction mixture was diluted with 1,2,4-trichlorobenzene (90 g) and filtered. The 4,4'-oxydiphthalic anhydride was allowed to crystallize and was collected, washed with 1,2,4-trichlorobenzene followed by hexane, and dried in an air circulating oven at 120° C to give crude 4,4'-oxydiphthalic anhydride, 11.4 g.

Example 27

4-Fluorophthalic anhydride (0.44 g, 2.6 mmol shown as FPAN in table below), potassium fluoride (Aldrich, 0.18 g, 3.1 mmol), benzoic acid (Aldrich, 0.28 g, 2.3 mmol shown as BzOH in table below) and dimethyl formamide (Aldrich, 3 ml) were heated in a 160° C oil bath. The reaction was monitored by GC and showed the following results (BOPAN = benzoyloxyphthalic anhydride; ODPAN = oxydiphthalic anhydride; and HOPAN = hydroxyphthalic anhydride):

GC Area Percent

| Time | FPAN | BzOH | BOPAN | ODPAN | HOPAN |
|---|---|---|---|---|---|
| 0.5 hrs. | 10.6 | 10.3 | 37.3 | 17.1 | 0.22 |
| 1.5 hrs. | 3.5 | 5.5 | 57.8 | 19.5 | 1.1 |

Example 28

4-Fluorophthalic anhydride (0.44 g, 2.6 mmol shown as FPAN in table below), potassium fluoride (Aldrich, 0.14 g, 2.4 mmol), benzoic acid (Aldrich, 0.31 g, 2.5 mmol shown az BzOH in table below) and N-methyl pyrrolidinone (Aldrich, 3 ml) were heated in a 160° C oil bath. The reaction was monitored by GC

and showed the following results (BOPAN = benzoyloxyphthalic anhydride; ODPAN = oxydiphthalic anhydride; and HOPAN = hydroxyphthalic anhydride):

GC Area Percent

| Time | FPAN | BzOH | BOPAN | ODPAN | HOPAN |
|---|---|---|---|---|---|
| 0.5 hrs. | 19.3 | 38.6 | 23.5 | 5.2 | 1.8 |
| 1.5 hrs. | 1.6 | 26.8 | 52.6 | 9.0 | 1.6 |

Example 29

3-Fluorophthalic anhydride (19.6 g, 88% pure, 104 mmol), potassium fluoride (7.6 g, 130 mmol), benzoic acid (12.2 g, 100 mmol), and dimethyl formamide (90 g) were heated to 130-135° C for 5 hours. An aliquot from the reaction was removed and showed 56% BOPAN and 16% ODPAN (GC area percent). The reaction mixture was filtered and the DMF removed under reduced pressure. Ethyl acetate (17 ml) was added and the suspension filtered to give ODPAN (4.1 g, 92% pure) and a filtrate. The filtrate was allowed to cool to room temperature and the solid removed by filtration, 12.3 g. Analysis of this solid by GC showed the presence of BOPAN (72%) and ODPAN (26%). Hexane was added to the filtrate and a new solid was formed which was collected, 12.2g. GC analysis of this sclid showed that it was 60% BOPAN.

Example 30

4-Bromophthalic anhydride (0.53 g, 2.3 mmol), potassium fluoride (0.20 g, 3.4 mmol), benzoic acid (0.32 g, 2.6 mmol), and dimethyl formamide (8.2 g) were heated to about 150° C. The reaction was monitored by GC and showed 5.2 ratio of BrPAN to BOPAN after 2.3 hours.

Example 31

4-Chlorophthalic anhydride (0.68 g, 3.7 mmol), benzoic acid (0.43 g, 3.5 mmol), potassium fluoride (0.37 g, 6.4 mmol), and DMF (6.88 g) were heated to about 150° C. The reaction was monitored by GC and showed a 5.4 ratio of CPAN to BOPAN after 4.5 hours.

Example 32

3-Bromophthalic anhydride (22.7 g, 100 mmol), potassium benzoate (Aldrich, 17.0 g, 106 mmol), and dimethyl formamide (114 g) were heated to 150° C. The reaction was monitored by GC. After 2.5 hours, GC analysis of the reaction mixture showed a 2.8:1 ratio of 3-benzoyloxyphthalic anhydride to 3-bromophthalic anhydride with smaller amounts of 3-hydroxyphthalic anhydride. The reaction mixture was cooled to 85° C and the DMF distilled under reduced pressure. Ethyl acetate (90 mls) was added and the reaction vessel heated to reflux. The solids were removed by filtration and the ethyl acetate layer allowed to cool and stand overnight. A slightly off-white solid was collected which contained 3-benzoyloxyphthalic anhydride (76% pure, 12.1 g).

Example 33

3-Bromophthalic anhydride (44.4 g, 196 mmol), potassium benzoate (Aldrich, 78.0 g, 487 mmol), and dimethyl formamide (184 g) were heated to 157° C. The reaction was monitored by GC and indicated the presence of 3-hydroxyphthalic anhydride (34 GC area percent), 3-benzoyloxyphthalic anhydride (12.5 GC area percent), and only a small amount of 3-bromophthalic anhydride (2.4 GC area percent).

Example 34

4-Chlorophthalic anhydride (10 g, 55 mmol), potassium benzoate (1.0 g, 6.2 mmol) was heated to 162° C in sulfolane (25 g). Potassium carbonate (3.87 g, 28 mmol) was added and the reaction heated to

175°C. After 4 hours, analysis of the reaction mixture by GC indicated the presence of 4-benzoyloxyphthalic anhydride (10 GC area percent), 4-hydroxyphthalic anhydride (7.7 GC area percent), and 4,4'-oxydiphthalic anhydride (71 GC area percent) along with unreacted 4-chlorophthalic anhydride (11.4 GC area percent).

Example 35

4-Chlorophthalic anhydride (10 g, 55 mmol), potassium benzoate (1.0 g, 6.2 mmol) were heated in DMF (25 g) to reflux. Potassium carbonate (3.87 g, 28 mmol) was added. After heating for 7.5 hours, GC analysis of the reaction mixture indicated the presence of unreacted starting material (19.7 GC area percent), 4-benzoyloxyphthalic anhydride (9.6 GC area percent), 4-hydroxyphthalic anhydride (17.0 GC area percent), and 4,4'-oxydiphthalic anhydride (52.9 GC area percent).

Example 36

4-Fluorophthalic anhydride (16.7 g, 100 mmol) and sodium benzoate (14.5 g, 101 mmol) were heated to about 145°C in DMF (70 ml). After heating for 5 hours, the solvent was distilled off under reduced pressure (the temperature was kept at less than 100°C). Ethyl acetate (50 ml) was added and the suspension refluxed for 30 minutes. The suspension was filtered and the ethyl acetate allowed to cool to give 4-benzoyloxyphthalic anhydride (13.6 g, 86% pure) as a whitish solid. Ethyl acetate (8 mls) were distilled from the mother liquor and hexane added to the hot solution. The solution was allowed to cool and stand for several days upon which a solid benzoyloxyphthalic anhydride precipitated (4.7 g, 79% pure).

Example 37

4-Fluorophthalic anhydride (0.40 g, 90% pure), potassium fluoride (0.20 g), and acetic acid (0.14 ml) were heated in DMF (5 ml) at 150°C for 30 minutes. The reaction mixture was analyzed by GC-MS which showed the presence of 4-acetoxyphthalic anhydride (14.8%) and 4-hydroxyphthalic anhydride (9.7%).

Example 38

4-Chlorophthalic anhydride (7.5 g, 41.1 mmol) and 1,2,4-trichlorobenzene (Aldrich, 7.5 g) were heated to reflux and dry potassium carbonate (0.55 g, 4.1 mmol) was added. After refluxing for 30 minutes, 4-(3-nitrobenzoyl)oxyphthalic anhydride (0.03 g, 0.1 mmol), tetraphenylphosphonium bromide (Hokko Chemical, 0.08 g, 0.2 mmol), and potassium carbonate (2.9 g, 21 mmol) were added. The reaction was heated for 7 hours and was then diluted with 1,2,4-trichlorobenzene (50 g), filtered, and the 4,4'-oxydiphthalic anhydride allowed to crystallize. The solid was collected, washed with solvent, and dried in an air circulating oven to give crude 4,4-oxydiphthalic anhydride, 3.9 g (61% isolated yield).

Example 39

4-Chlorophthalic anhydride (7.5 g, 41.1 mmol) and 1,2,4-trichlorobenzene (Aldrich, 7.5 g) were heated to reflux and dry potassium carbonate (0.6 g, 4.3 mmol) was added. After refluxing for 30 minutes, 4-benzoyloxyphthalic anhydride (0.024 g, 0.1 mmol), tetraphenylphosphonium bromide (Hokko Chemical, 0.08 g, 0.2 mmol), and potassium carbonate (2.9 g, 21 mmol) were added. The reaction was heated for 7 hours and was then diluted with 1,2,4-trichlorobenzene (50 g), filtered, and the 4,4'-oxydiphthalic anhydride allowed to crystallize. The solid was collected, washed with solvent, and dried in an air circulating oven to give crude 4,4-oxydiphthalic anhydride, 3.56 g (56% isolated yield).

Example 40

4-Chlorophthalic anhydride (7.5 g, 41.1 mmol) and 1,2,4-trichlorobenzene (Aldrich, 7.5 g) were heated to reflux and dry potassium carbonate (0.6 g, 4.3 mmol) was added. After refluxing for 30 minutes, 4-(4-methoxybenzyol)oxyphthalic anhydride (0.04 g, 0.1 mmol), tetraphenylphosphonium bromide (Hokko Chemical, 0.08 g, 0.2 mmol), and potassium carbonate (2.84 g, 20.5 mmol) were added. The reaction was heated for 6.5 hours and was then diluted with 1,2,4-trichlorobenzene (30 g), filtered, and the 4,4'-oxydiphthalic anhydride allowed to crystallize. The solid was collected, washed with solvent, and dried in an air circulating oven to give crude 4,4-oxydiphthalic anhydride, 0.62 g (10% isolated yield).

Example 41

4-Chlorophthalic anhydride (7.5 g, 41.1 mmol) and 1,2,4-trichlorobenzene (Aldrich, 7.5 g) were heated to reflux and dry potassium carbonate (0.6 g, 4.3 mmol) was added. After refluxing for 30 minutes, 4-(4-methylbenzoyl)oxyphthalic anhydride (0.029 g, 0.10 mmol), tetraphenylphosphonium bromide (Hokko Chemical, 0.08 g, 0.2 mmol), and potassium carbonate (2.9 g, 21 mmol) were added. The reaction was heated for 10 hours and was then diluted with 1,2,4-trichlorobenzene (30 g), filtered, and the 4,4'-oxydiphthalic anhydride allowed to crystallize. The solid was collected, washed with solvent, and dried in an air circulating oven to give crude 4,4-oxydiphthalic anhydride, 1.53 g (24% isolated yield).

Example 42

4-Fluorophthalic anhydride (16.7 g, 100 mmol), 4-(4-chlorobenzoyl)oxyphthalic anhydride (0.32 g, 1.1 mmol), and potassium fluoride (14.4 g, 250 mmol) were suspended in DMF (50 ml) and heated and maintained between 130-135° C. Water (9 drops) was added after the reaction mixture had been heated for 90 minutes and again after heating for 6 hours. The reaction was monitored by GC and showed a 10% conversion to ODPAN after 4 hours. The reaction mixture was heated for a total of 12 hours. The solvent was distilled under reduced pressure (temperature of less than 100° C). The residue was treated with 1N potassium hydroxide followed by 40% potassium hydroxide until all solids had dissolved and the pH of the solution was 13. The mixture was then acidified with concentrated hydrochloric acid until the pH of the suspension was 0.8. The suspension was allowed to stir overnight and then the solid was collected, washed, and dried to give 4,4'-oxydiphthalic acid as a white solid, 16.2 g (88% pure).

Comparative Example 1

4-Chlorophthalic anhydride (30 g, 160 mmol) and 1,2,4-trichlorobenzene (60 g) were heated to reflux (approximately 224° C) and tetraphenylphosphonium bromide (Hokko Chemical, 0.30 g, 0.7 mmol) was added. Dry potassium carbonate (7.95 g, 58 mmol) was then added in portions over one hour. The reaction was monitored by GC and showed only 2.5% conversion to 4,4'-oxydiphthalic anhydride (ODPAN) 3 hours after addition of carbonate was completed.

Comparative Example 2

4-Chlorophthalic anhydride (7.5 g, 41.1 mmol) and 1,2,4-trichlorobenzene (Aldrich, 7.5 g) were heated to reflux and dry potassium carbonate (0.7 g, 5.1 mmol) was added. After refluxing for 30 minutes, tetraphenylphosphonium bromide (Hokko Chemical 0.075 g, 0.2 mmol), and potassium carbonate (2.9 g, 21 mmol) were added. The reaction was heated for 22.5 hours and was then diluted with 1,2,4-trichlorobenzene (22 g), filtered, and the 4,4'-oxydiphthalic anhydride allowed to crystallize. The solid was collected, washed with solvent, and dried in an air circulating oven to give crude 4,4-oxydiphthalic anhydride, 0.6 g (10% isolated yield).

**Claims**

1. A process for the preparation of acyloxyphthalic anhydrides which comprises mixing a halophthalic anhydride with an effective alkali metal salt, and an effective acid or acid derivative to form a mixture and heating said mixture.

2. A process according to claim 1 which further comprises converting said acyloxyphthalic anhydride to oxydiphthalic anhydride by reacting said acyloxyphthalic anhydride with a halophthalic anhydride in the presence of an effective alkali metal salt.

3. A process for the preparation of oxydiphthalic anhydride, and acyloxyphthalic anhydrides which comprises reacting a halophthalic anhydride with an alkali metal salt and at least a catalytically effective amount of an acid or acid derivative.

4. A process according to claim 3, wherein the product is predominantly oxydiphthalic anhydride; said process comprising reacting a halophthalic anhydride with an alkali metal salt and a catalytically effective amount of an acid or acid derivative.

5. A process according to any of claims 1 to 4 wherein said acid or acid derivative is benzoic acid, a substituted benzoic acid, a benzoic acids salt, a salt of a substituted benzoic acid, a hydrolyzable benzoyl ester, a hydrolyzable substituted benzoyl ester, an alkyl carboxylic acid, a hydrolyzable ester of an alkyl carboxylic acid, a salt of an alkyl carboxylic acid or mixtures thereof.

6. A process according to claim 3 wherein the product is predominantly an acyloxyphthalic anhydride; said process comprising reacting a halophthalic anhydride with an alkali metal salt and an acid reactant.

7. A process according to claim 6 wherein the acid reactant is benzoic acid, a substituted benzoic acid, a benzoic acids salt, a salt of a substituted benzoic acid, an alkyl carboxylic acid, a salt of an alkyl carboxylic acid or mixtures thereof.

8. A process according to any of claims 1 to 7 wherein said alkali metal salt is potassium carbonate, sodium carbonate, potassium fluoride and/or cesium fluoride.

9. A process according to any of claims 1 to 8 wherein said mixture is heated to a temperature between about 120 and 275°C.

10. A process according to any of claims 1 to 9 wherein the process is conducted in a solvent.

11. A process according to claim 10 wherein said solvent is dimethyl formamide, N-methyl pyrrolidone, dimethyl acetamide, hexamethylphosphoramide, dimethyl sulfoxide, sulfolane, dimethyl sulphone, chlorobenzene, dichlorobenzene, trichlorobenzene and/or benzonitrile.

12. An acyloxyphthalic anhydride of the formula

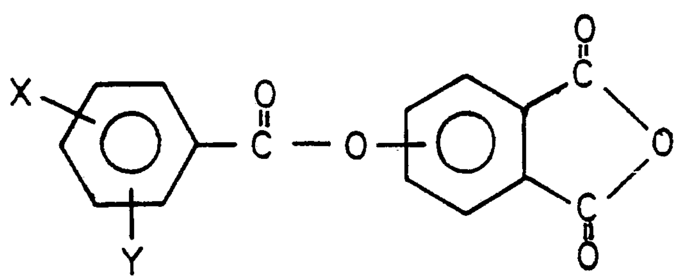

where X and Y are substituents independently selected from H, F, Cl, Br, $NO_2$, $CH_3$, $CF_3$, $CCl_3$ and CN, provided that X and Y may not both be H.

13. An acyloxyphthalic anhydride according to claim 12 namely 3-(4-chlorobenzoyloxy)phthalic anhydride,

4-(4-chlorobenzoyloxy)phthalic anhydride,

4-(3-chlorobenzoyloxy)phthalic anhydride,

4-(2-chlorobenzoyloxy)phthalic anhydride,

4-(3-bromobenzoyloxy)phthalic anhydride,

4-(2,4-dichlorobenzoyloxy)phthalic anhydride,

4-(2,5-dichlorobenzoyloxy)phthalic anhydride,

4-(2,6-dichlorobenzoyloxy)phthalic anhydride,

4-(3,4-dichlorobenzoyloxy)phthalic anhydride,

4-(3,5-dichlorobenzoyloxy)phthalic anhydride,

4-(3-methoxybenzoyloxy)phthalic anhydride,

4-(4-methylbenzoyloxy)phthalic anhydride,

4-(3-nitrobenzoyloxy)phthalic anhydride,

4-(4-nitrobenzoyloxy)phthalic anhydride,

4-(2-(trifluormethyl)benzoyloxy)phthalic anhydride,

4-(3-(trifluormethyl)benzoyloxy)phthalic anhydride, and

4-(4-(trifluormethyl)benzoyloxy)phthalic anhydride.

**14.** An acyloxyphthalic anhydride of the formula:

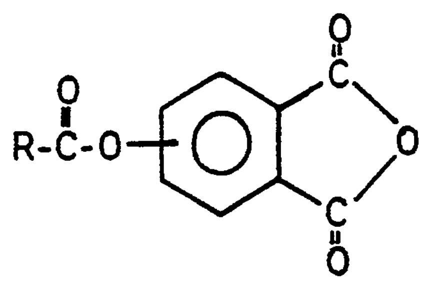

where R is $C_2H_5$, $C_3H_7$, $C_4H_9$, $C_5H_{11}$, $C_6H_{13}$, $C_7H_{15}$, $C_8H_{17}$, $C_9H_{19}$, $C_{10}H_{21}$ or $C_{11}H_{23}$, including branched and cyclic isomers of those formulas.

**15.** Use of the acyloxyphthalic anhydride of claims 12 and 13 and those obtained by the processes according to any of claims 1 to 11 for the synthesis of oxydiphthalic anhydrides or as curing agent for epoxy resins.

**16.** Use of the oxydiphthalic anhydrides obtained by the processes according to any of claims 2 to 11 for the preparation of polyimide resins.

**Claims for the following Contracting State: ES**

**1.** A process for the preparation of acyloxyphthalic anhydrides which comprises mixing a halophthalic anhydride with an effective alkali metal salt, and an effective acid or acid derivative to form a mixture and heating said mixture.

**2.** A process according to claim 1 which further comprises converting said acyloxyphthalic anhydride to oxydiphthalic anhydride by reacting said acyloxyphthalic anhydride with a halophthalic anhydride in the presence of an effective alkali metal salt.

**3.** A process for the preparation of oxydiphthalic anhydride and acyloxyphthalic anhydrides which comprises reacting a halophthalic anhydride with an alkali metal salt and at least a catalytically effective amount of an acid or acid derivative.

**4.** A process according to claim 3, wherein the product is predominantly oxydiphthalic anhydride; said process comprising reacting a halophthalic anhydride with an alkali metal salt and a catalytically effective amount of an acid or acid derivative.

**5.** A process according to any of claims 1 to 4 wherein said acid or acid derivative is benzoic acid, a substituted benzoic acid, a benzoic acids salt, a salt of a substituted benzoic acid, a hydrolyzable

benzoyl ester, a hydrolyzable substituted benzoyl ester, an alkyl carboxylic acid, a hydrolyzable ester of an alkyl carboxylic acid, a salt of an alkyl carboxylic acid or mixtures thereof.

6. A process according to claim 3 wherein the product is predominantly an acyloxyphthalic anhydride; said process comprising reacting a halophthalic anhydride with an alkali metal salt and an acid reactant.

7. A process according to claim 6 wherein the acid reactant is benzoic acid, a substituted benzoic acid, a benzoic acids salt, a salt of a substituted benzoic acid, an alkyl carboxylic acid, a salt of an alkyl carboxylic acid or mixtures thereof.

8. A process according to any of claims 1 to 7 wherein said alkali metal salt is potassium carbonate, sodium carbonate, potassium fluoride and/or cesium fluoride.

9. A process according to any of claims 1 to 8 wherein said mixture is heated to a temperature between about 120 and 275 °C.

10. A process according to any of claims 1 to 9 wherein the process is conducted in a solvent.

11. A process according to claim 10 wherein said solvent is dimethyl formamide, N-methyl pyrrolidone, dimethyl acetamide, hexamethylphosphoramide, dimethyl sulfoxide, sulfolane, dimethyl sulphone, chlorobenzene, dichlorobenzene, trichlorobenzene and/or benzonitrile.

12. Use of the acyloxyphthalic anhydride obtained by the processes according to any of claims 1 to 11 for the synthesis of oxydiphthalic anhydrides or as curing agent for epoxy resins.

13. Use of the oxydiphthalic anhydrides obtained by the processes according to any of claims 2 to 11 for the preparation of polyimide resins.